# EUROPÄISCHE PATENTSCHRIFT

(11) **EP 1 531 155 B1**
(45) Veröffentlichungstag und Bekanntmachung des Hinweises auf die Patenterteilung: **01.08.2007**
(21) Anmeldenummer: 04025730.5
(22) Anmeldetag: 29.10.2004
(51) Int. Cl.: C07F 7/18, C07F 7/08, C07D 333/18, H01L 29/24

(54) **Unsymmetrische lineare organische Oligomere**
Asymmetrical linear organic oligomers
Oligomères organiques linéaires asymétriques

(30) Priorität: 12.11.2003 DE 10353093
(43) Veröffentlichungstag der Anmeldung: 18.05.2005
(73) Patentinhaber: H.C. Starck GmbH & Co. KG, 38642 Goslar (DE)
(72) Erfinder: Kirchmeyer, Stephan, Dr., 51375 Leverkusen (DE); Ponomarenko, Sergei, Dr., 107589 Moskau (RU); Halik, Marcus, Dr., 91058 Erlangen (DE)
(74) Vertreter: Clauswitz, Kai-Uwe Wolfram

(56) Entgegenhaltungen:
- US-A1- 2004 183 069
- US-B1- 6 414 164
- DATABASE CA [Online] CHEMICAL ABSTRACTS SERVICE, COLUMBUS, OHIO, US; PARK, MI-KYOUNG ET AL: "Synthesis of monofunctional and amphiphilic oligothiophenes" XP002316608 gefunden im STN Database accession no. 130:352677 & POLYMERIC MATERIALS SCIENCE AND ENGINEERING , 80, 241-242 CODEN: PMSEDG; ISSN: 0743-0515, 1999,
- DATABASE CA [Online] CHEMICAL ABSTRACTS SERVICE, COLUMBUS, OHIO, US; ADVINCULA R. C. ET AL: "Towards the synthesis of mono- and bi-functional amphiphilic oligothiophenes: organic materials for opto-electronic applications" XP002316609 gefunden im STN Database accession no. 131:351181 & MATERIALS RESEARCH SOCIETY SYMPOSIUM PROCEEDINGS, Bd. 561, 1999, Seiten 155-160, ISSN: 0272-9172
- MICHALITSCH, R. ET AL: "A practical synthesis of functionalized alkyl-oligothiophenes for molecular self-assembly" JOURNAL OF HETEROCYCLIC CHEMISTRY , 38(3), 649-653 CODEN: JHTCAD; ISSN: 0022-152X, 2001, XP002316604
- KATZ, HOWARD E. ET AL: "Synthesis, Solubility, and Field-Effect Mobility of Elongated and Oxa-Substituted .alpha.,.omega.-Dialkyl Thiophene Oligomers. Extension of "Polar Intermediate" Synthetic Strategy and Solution Deposition on Transistor Substrates" CHEMISTRY OF MATERIALS , 10(2), 633-638 CODEN: CMATEX; ISSN: 0897-4756, 1998, XP002316605
- AFZALI, A. ET AL: "An Efficient Synthesis of Symmetrical Oligothiophenes: Synthesis and Transport Properties of a Soluble Sexithiophene Derivative" CHEMISTRY OF MATERIALS , 14(4), 1742-1746 CODEN: CMATEX; ISSN: 0897-4756, 2002, XP002316606
- ARAKI, YASUYUKI ET AL: "Photoinduced Charge Separation and Charge Recombination of Oligothiophene-Viologen Dyads in Polar Solvent" JOURNAL OF PHYSICAL CHEMISTRY A , 108(48), 10649-10655 CODEN: JPCAFH; ISSN: 1089-5639, 2004, XP002316607

## Beschreibung

Die Erfindung betrifft unsymmetrische lineare organische Oligomere, ein Verfahren zu deren Herstellung sowie ihre Verwendung als Halbleiter in elektronischen Bauelementen.

Das Feld molekularer Elektronik hat sich in den letzten 15 Jahren mit der Entdeckung organischer leitender und halbleitender Verbindungen rapide entwickelt. In dieser Zeit wurden eine Vielzahl von Verbindungen gefunden, die halbleitende oder elektrooptische Eigenschaften aufweisen. Halbleitende organische Verbindungen werden derzeit für Anwendungsgebiete wie organische FeldEffekt-Transistoren (OFET's), organische Lumineszenzdioden (OLED's), Sensoren und photovoltaische Elemente entwickelt.

Ein Feldeffekttransistor ist ein Dreielektroden-Element, in dem die Leitfähigkeit eines dünnen Leitungskanals zwischen zwei Elektroden (genannt "Source" und "Drain") mittels einer an der dritten, durch eine dünne Isolatorschicht von dem Leitungskanal getrennten, Elektrode (genannt "Gate") kontrolliert wird. Die wichtigsten charakteristischen Eigenschaften eines FeldeffektTransistors sind die Mobilität der Ladungsträger, die entscheidend die Schaltgeschwindigkeit des Transistors bestimmt, und das Verhältnis zwischen den Strömen im geschalteten und ungeschalteten Zustand, das sogenannte "On/Off-ratio". Eine weitere wichtige Eigenschaft eines Feldeffekt-Transistors ist die Einsatzspannung bei der ein messbarer Strom zwischen Source und Drain zu fließen beginnt. Diese Spannung wird auch Schwellenspannung genannt. Es besteht generell der Wunsch nach niedrigen Schwellenspannungen. Um diese Schwellenspannung zu vermindern, versucht man die Isolatorschicht zwischen Gate und dem Leitungskanal so dünn wie möglich zu gestalten.

Die dünnst möglichen Isolatorschichten können aus sogenannten "selbstorganisierenden Monoschichten", auch Self Assembled Monolayers (SAM) genannt, hergestellt werden. Geeignete Moleküle, aus denen SAM-Schichten hergestellt werden können, sind beispielsweise lange lineare Alkane mit mehr als 10 Kohlenstoffatomen, die über geeignete funktionelle Gruppen des Moleküls auf dem Trägermaterial des Transistors verankert werden können. US 6,433,359 B1 beschreibt die Verwendung von linearen, verzweigten oder cycloaliphatischen Kohlenwasserstoffen mit polaren Gruppen, wie beispielsweise Chlorsilyl-, Carboxy-, Hydroxy-, Amino-, Amido- und Thiolgruppen zur Herstellung von Feldeffekt-Transistoren, die SAM-Schichten zwischen Isolatorschicht und Halbleiterschicht enthalten um "On/Off ratio", Schwellenspannung (Theshold Voltage, d.h. die am Gate anliegende Spannung, bei der zwischen Source und Drain ein meßbarer Strom zu fließen beginnt), und die Mobilität der Ladungsträger zu verbessern. Die Verwendung von Thiolgruppen enthaltenden SAM-Schichten mit ähnlicher Zielausrichtung ist in US 6,335,539 B1 beschrieben.

Die besten Eigenschaften hat man jedoch in Feldeffekt-Transistoren gefunden, in denen aus SAM-Molekülen aufgebaute Schichten als eigene Isolatorschicht verwendet werden. So wurden beispielsweise Feldeffekt-Transistoren, die bei einer Schwellenspannung von 1-2 V einen Strom zwischen Source und Drain zeigen, mit einer ca. 2 nm dünnen SAM-Isolatorschicht aus Alkyltrichlorosilanen hergestellt (J. Collet et al., Appl. Phys. Lett. 1998, Vol. 73, No. 18, 2681-2683 und Appl. Phys. Lett. 2000, Vol. 76, No. 14, 1941-1943.

Nachteilig bei alkylhaltigen SAM-Schichten ist jedoch, dass sie schwer zu strukturieren sind bzw. es aufgrund der niedrigen Oberflächenspannung von ca. 20-30 mN/m Schwierigkeiten bereitet, weitere Schichten z.B. durch Nassverfahren auf diese Schichten aufzutragen. Durch Funktionalisierungen, beispielsweise eines Endes der SAM-Moleküle z.B. durch Carboxylgruppen, konnte die Oberflächenspannung auf ca. 50 mN/m vergrößert werden (Appl. Phys. Lett. 2000, Vol. 76, No. 14. S. 1941-1943).

Auch die Anknüpfung der Alkyl(en)ketten der SAM-Moleküle an halbleitende, konjugierte Oligomere, wie beispielweise Oligothiophene, ermöglichte einerseits eine Optimierung der Oberflächenspannung zwischen Isolatorschicht und Halbleiterschicht und andererseits einen geordneten Aufbau zweier Schichten, der dielektrischen und der halbleitenden Schicht, in einem Schritt. Zwar wurden bereits solche SAM-Moleküle mit Oligothiophenen an einem und Thiolgruppen am anderen Ende einer Alkylengruppe beschrieben, wie z.B. 12-(2,2':5',5",2"'-Quaterthien-5-yl)dodecanthiol (Colloids Surf. A, 198-200 (2002) 577-591) oder 11-(2,2':5',2"-Terthien-5-yl)undec-1-ylthiol (Bäuerle et al., J. Phys. Chem. B 1997, Vol. 101, No. 31, 5951-5962), jedoch weisen thiolgruppenhaltige Verbindungen bei der Erzeugung von SAM-Schichten bekanntermaßen den Nachteil auf, dass diese sich lediglich auf Goldoberflächen verankern lassen.

Polare Gruppen-enthaltende Thiophene sind beschrieben in US 6414164B1

Berlin und Zotti et al. (J. Am. Chem. Soc. 1998, B.120, S.13453-13460) beschreiben carboxyalkylsubstituierte Di- und Terthiophene, die geeignet sind, Monoschichten auf Indium-Zinn-OxidSchichten (ITO) auszubilden. Der Nachteil dieser Verbindungen besteht jedoch darin, dass Di- und Terthiophene keine halbleitenden Eigenschaften aufweisen und sich lediglich als Zwischenstufen für weitere Umsetzungen eignen. So wurden beispielsweise Di- bzw. Terthiophene oxidativ nach Aufbringung als Schicht polymerisiert, jedoch wurden dadurch dotierte leitfähige Polymere erhalten, die keinen Halbleitereffekt aufweisen. Somit sind die dort beschriebenen Moleküle sowohl vor und als auch nach Polymerisation als Halbleiter ungeeignet.

Es wurden weitere mit jeweils einem Thiophen-, Pyrrol- oder sonstigem aromatischen Ring am Ende des Moleküls funktionalisierten SAM-Verbindungen, wie z.B. 11-(3-Thienyl)undecyl-trichlorosilan (Mat. Res. Soc. Symp. Proc. 2002, B.708, S.305-309) oder ähnliche Verbindungen (US-A 2003/0099845) beschrieben. Diese Verbindungen zeigen aber ebenfalls keine halbleitenden Eigenschaften. Nach oxidativer Polymerisation bilden sie elektrisch leitfähige, auf dem Substrat verankerte Polymerschichten, wie z.B. Polypyrrol-, Polythiophen-, Polyacetylen- und Polydiacetylen-Schichten, die jedoch in der oxidierten, elektrisch leitfähigen und nicht in der neutralen, halbleitenden Form vorliegen.

Es besteht daher weiterhin auf Bedarf an SAM-Molekülen, die zur Bildung von dünnen Isolatorschichten auf geeigneten Substraten und gleichzeitig zur Bildung von halbleitenden Schichten geeignet sind.

Überraschend wurde nun gefunden, dass halbleitende Oligomere, die eine mit einer geeigneten polaren Gruppe substituierte lineare Alkylenkette tragen, hierfür in besonderem Maße geeignet sind.

Unter Oligomeren sind im Rahmen der Erfindung Verbindungen mit 2 bis 15, bevorzugt 3 bis 13, besonders bevorzugt 4 bis 10 Monomereinheiten (im Folgenden Ar-Einheiten), die gleich oder verschieden sein können, zu verstehen.

Gegenstand der vorliegenden Erfindung sind Verbindungen der allgemeinen Formel (I), 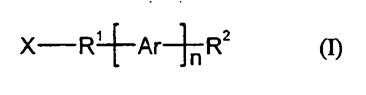 worin
- n: eine ganze Zahl von 4 bis 10 ist,
- Ar: unabhängig für jedes n für einen gegebenenfalls substituierten 1,4-Phenylen, 2,7-Fluorenylen-, 2,5-Thienylen- oder 1,2-Ethenylenrest steht, wobei wenigstens ein gegebenenfalls substituierter 2,5-Thienylenrest oder gegebenenfalls substituierter 2,5-Thienylenrest und gegebenenfalls substituierter 1,4-Phenylenrest enthalten ist,
- R¹: für eine gegebenenfalls durch ein(e) oder mehrere O- oder S-Atome, Silylen-, Phosphonoyl- oder Phosphorylgruppen unterbrochene C₃-C₃₀-Alkylengruppe steht,
- R²: für H oder eine lineare oder verzweigte C₁-C₂₀-Alkylgruppe, bevorzugt eine C₁-C₁₂-Alkylgruppe, oder eine gegebenenfalls durch ein(e) oder mehrere O- oder S-Atome, Silylen-, Phosphonoyl- oder Phosphorylgruppen unterbrochene lineare C₁-C₂₀-Alkylgruppe, bevorzugt C₁-C₁₂-Alkylgruppe, steht und
- X: für eine Gruppe ausgewählt aus einer gegebenenfalls substituierten Vinyl-, Alkoxysilyl-, Chlorosilyl- oder Siloxangruppe steht.

Erfindungsgemäß können die n Ar-Einheiten in der allgemeinen Formel (I) gleich oder verschieden und in beliebiger Reihenfolge miteinander verknüpft sein. Die n Ar-Einheiten bilden eine konjugierte Oligomerkette. In bevorzugten Ausführungsformen kann Ar neben 1,4-Phenylen, 2,7-Fluorenylen und/oder 2,5-Thienylen auch für 1,2-Ethenylen-Einheiten stehen, wodurch die Konjugationslänge nicht verkürzt wird. Bevorzugt enthalten die erfindungsgemäßen Verbindungen der allgemeinen Formel (I) für Ar gegebenenfalls substituierte 2,5-Thienyleneinheiten oder gegebenenfalls substituierte 2,5-Thienyleneinheiten und gegebenenfalls substituierte 1,4-Phenyleneinheiten, wobei die gegebenenfalls vorhandenen Substituenten gleich oder verschieden sein können. Für den Fall, dass gegebenenfalls substituierte 2,5-Thienyleneinheiten und gegebenenfalls substituierte 1,4-Phenyleneinheiten enthalten sind, sind solche neuen Verbindungen besonders bevorzugt, die keine gegebenenfalls substituierten 1,4-Phenyleneinheiten benachbart enthalten. Ganz besonderes bevorzugt sind Verbindungen der allgemeinen Formel (I) die für Ar lediglich 2,5-Thienyleneinheiten enthalten.

Als Substituenten für Ar kommen beispielsweise lineare oder verzweigte C₁-C₂₀-Alkylreste, bevorzugt C₁-C₁₂-Alkylreste; oder durch ein(e) oder mehrere O-Atome unterbrochene lineare C₁-C₂₀-Alkylreste in Frage. An den 2,7-Fluorenyleneinheiten sitzen gegebenenfalls vorhandene Substituenten (einer oder mehrere, bevorzugt zwei) bevorzugt an der 9-Position. Besonderes bevorzugte Substituenten für 2,5-Thienylen- oder 1,4-Phenylen-Einheiten sind Methyl-, Ethyl-, Propyl-, Buthyl-, Pentyl- oder Hexyl-Gruppen. Weitere besonderes bevorzugte Substituenten für 1,4-Phenylen-Einheiten sind Methoxy-, Ethoxy-, Propoxy-, Buthoxy-, Pentoxy-, Hexyloxy- oder Heptyloxy-Gruppen. Ganz besonderes bevorzugter Substituent ist Wasserstoff.

Beispielhaft für bevorzugte Reste R¹ seien Decylen-, Undecylen, Dodecylen, Terdecylen-, Hexadecylen- oder Octadecylengruppen genannt. Beispielhaft für bevorzugte R² seien Methyl-, Ethyl-, Propyl-, Butyl-, Pentyl-, Hexyl-, Heptyl-, Octyl-, Nonyl-, Decyl-, Undecyl- oder Dodecylgruppen genannt. Ganz besonderes bevorzugt sind Ethyl- oder Hexylgruppen.

X in der allgemeinen Formel (I) steht für eine Gruppe ausgewählt aus einer gegebenenfalls substituierte Vinylgruppen, Alkoxysilyl-, Chlorosilyl-, oder Siloxangruppe Diese Gruppen sind polar, so dass sie mit dem Substrat in chemische oder physikalische Wechselwirkung treten können bzw. können auf leichte Weise in solche überführt werden Alkoxysilylgruppen können im Rahmen der Erfindung Mono-, Di- oder Tri-C₁-C₂₀-alkoxysilylgruppen und Siloxangruppen Di-, Oligo- oder Polysiloxane aufgebaut aus mono-, di- oder trifunktionellen Einheiten sein. Ganz besonderes bevorzugt sind Vinyl-, Trimethoxysilyl-, Triethoxysilylgruppen oder Alkyldisiloxangruppen, wie beispielsweise eine Tetraalkyldisiloxangruppe,.

Bevorzugte neue Verbindungen der allgemeinen Formel (I) sind solche der allgemeinen Formel (I-a), 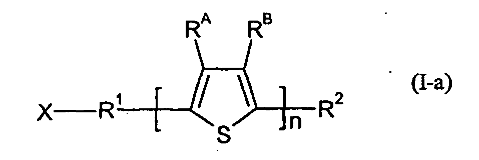 worin
- R^{A}, R^{B}: unabhängig voneinander jeweils für H oder eine gegebenenfalls substituierte lineare oder verzweigte gegebenenfalls durch 1 bis 5 Sauerstoffatome unterbrochene C₁-C₂₀-Alkylgruppe, bevorzugt für eine C₁-C₁₂-Alkylgruppe, besonders bevorzugt für eine Methyl-, Ethyl-, Propyl-, Butyl-, Pentyl- oder Hexylgruppe, eine gegebenenfalls substituierte C₁-C₂₀-Alkoxygruppe, bevorzugt für eine C₁-C₆-Alkoxygruppe, besonders bevorzugt für eine Methoxygruppe, oder gemeinsam für eine gegebenenfalls substituierte C₁-C₆-Dioxyalkylengruppe, bevorzugt für eine 3,4-Ethylendioxygruppe, stehen. und
n, X, R¹ und R² die oben für die allgemeine Formel (I) genannte Bedeutung haben.

In bevorzugten Ausführungsformen der vorliegenden Erfindung stehen R^{A} und R^{B} für H.

Weiterhin bevorzugte neue Verbindungen der allgemeinen Formel (I) sind solche, worin X für eine Vinylgruppe steht.

In bevorzugten Ausführungsformen der vorliegenden Erfindung sind dies solche Verbindungen der allgemeinen Formel (I-a-1), 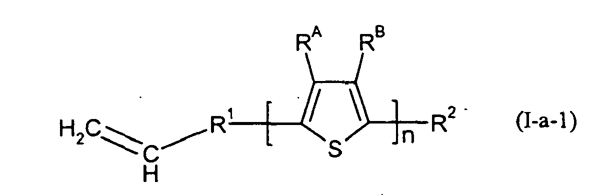 worin n, X, R¹ und R² die oben für die allgemeine Formel (I) genannte Bedeutung und R^{A} und R^{B} die oben für die allgemeine Formel (I-a) genannte Bedeutung haben.

Weiterhin bevorzugte neue Verbindungen der allgemeinen Formel (I) sind solche, worin X für eine Alkoxysilylgruppe, inbesondere eine Mono-, Di- oder Tri-C₁-C₂₀-alkoxysilylgruppe, wie z.B. Trimethoxysilyl oder Triethoxysilyl, oder eine Siloxangruppe, insbesondere eine Tetraalkyldisiloxangruppe, steht.

In einer weiteren bevorzugten Ausführungsform der Erfindung sind Verbindungen der allgemeinen Formel (I), worin X für eine Alkoxysilylgruppe steht, Verbindungen der allgemeinen Formeln (I-a-2), 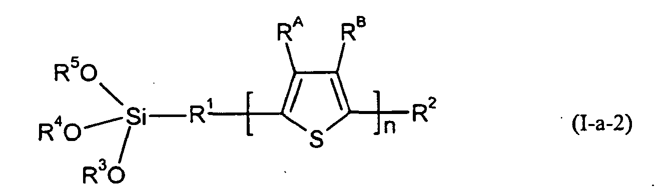 worin
- R³, R⁴ und R⁵: unabhängig voneinander für H oder eine lineare oder verzweigte C₁-C₂₀-Alkylgruppe, bevorzugt C₁-C₆-Alkylgruppe, besonders bevorzugt Methyl oder Ethyl, stehen und
n, R¹ und R² die oben für die allgemeine Formel (I) genannte Bedeutung und R^{A} und R^{B} die oben für die allgemeine Formel (I-a) genannte Bedeutung haben.

In bevorzugten Ausführungsformen der vorliegenden Erfindung sind Verbindungen der allgemeinen Formel (I), worin X für eine Siloxangruppe steht, Verbindungen der allgemeinen Formeln (I-a-3), 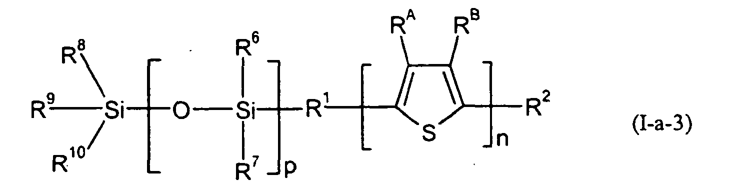 worin
- R⁶ bis R¹⁰: unabhängig voneinander für H oder eine lineare oder verzweigte C₁-C₂₀-Alkylgruppe, bevorzugt C₁-C₆-Alkylgruppe, besonders bevorzugt Methyl oder Ethyl, stehen,
- p: für eine ganze Zahl von 1 bis 5, bevorzugt für 2 oder 3, besonders bevorzugt für 2 steht und
n, R¹ und R² die oben für die allgemeine Formel (I) genannte Bedeutung und R^{A} und R^{B} die oben für die allgemeine Formel (I-a) genannte Bedeutung haben.

Prinzipiell ist es möglich, die erfindungsgemäßen Verbindungen mittels unterschiedlicher, dem Fachmann grundsätzlich bekannter Verfahren herzustellen, beispielsweise durch Kupplung von Grignard-Verbindungen mit Monohalogenarylverbindungen in Anwesenheit von Nickel- (Synthesis 1993, S. 1099-1103) oder Palladiumkatalysatoren (Chem. Mater. 1993, B.5, S. 430-436). Diese Synthesemethoden führen jedoch zu Verbindungen, die nur schwer und unter hohem Aufwand gereinigt werden können, weshalb die resultierenden Endprodukte für den Einsatz als Halbleiter schlecht geeignet sind.

Bevorzugt werden die erfindungsgemäßen Verbindungen mittels einer Variante der Suzuki-Kupplung, häufig auch als Suzuki-Kondensation bezeichnet, hergestellt. Die Suzuki-Kondensation bzw. Suzuki-Kupplung, d.h. die Umsetzung von Arylhalogeniden und Arylboronsäure-Verbindungen mit einer Pd-Verbidnung als Katalysator in Gegenwart einer Base, ist z.B. in Suzuki et al., Chem. Rev. 1995, 95, 2457-2483 beschrieben. In einer bevorzugten Ausführungsform wird das Verfahren gemäß einer Variante dieser Suzuki-Kupplung durchgeführt, wobei Aryl- bzw. Heteroarylhalogenide und Thiophen-Pinakolinboronsäurester gegebenenfalls in Anwesenheit wenigstens einer Base und/oder wenigstens eines Katalysators, der ein Metall der VIII. Nebengruppe des Periodensystems der Elemente, im Folgenden kurz als Metall der VIII. Nebengruppe bezeichnet, enthält, umgesetzt werden.

Ebenfalls Gegenstand der vorliegenden Erfindung ist daher ein Verfahren, bei dem eine bororganische Verbindung mittels Suzuki-Kupplung mit einem Aryl- bzw. Heteroarylhalogenid umgesetzt wird.

Bevorzugt ist dies ein Verfahren, bei dem als bororganische Verbindung eine Verbindung der allgemeinen Formel (II), 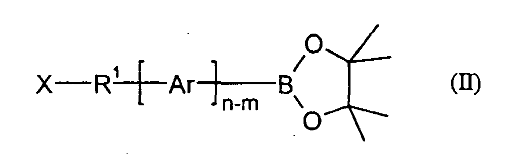 worin n, Ar, R¹ und X die oben für die allgemeine Formel (I) genannte Bedeutung haben und m für eine ganze Zahl von 1 bis 5, bevorzugt 1 bis 4, besonders bevorzugt 2, 3, oder 4 steht,
und als Aryl- bzw. Heteroarylhalogenid eine Verbindung der allgemeinen Formel (III), 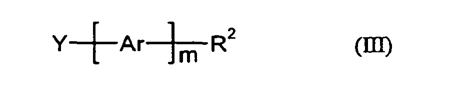 worin m und Ar die für die allgemeine Formel (II) und R² die oben für die allgemeine Formel (I) genannte Bedeutung haben und Y für Cl, Br, I oder -O-SO₂-R³, wobei R³ für eine Methyl-, Trifluormethyl-, Phenyl- oder Tolyl-Gruppe steht,
eingesetzt und diese miteinander umgesetzt werden.

Ganz besonders bevorzugt wird diese Verfahrensvariante zur Herstellung von neuen Verbindungen der allgemeinen Formel (I-a-1) angewendet, die anschließend durch weitere Modifizierung an der Doppelbindung zu anderen neuen Verbindungen der allgemeinen Formel (I-a), bevorzugt zu solchen der allgemeinen Formeln (I-a-2) oder (I-a-3) umgesetzt werden können.

Die bevorzugte Verfahrensvariante zur Herstellung der neuen Verbindungen (Suzuki-Kupplung) wird bei einer Temperatur von +20°C bis +200°C, bevorzugt von +40°C bis +150°C, besonderes bevorzugt von +80°C bis +130°C, in einem organischen Lösungsmittel oder Lösungsmittelgemisch durchgeführt.

Als Katalysatoren, die ein Metall der VIII. Nebengruppe enthalten, kommen prinzipiell alle geeigneten Verbindungen in Frage, die ein Metall der VIII. Nebengruppe, bevorzugt Pd, Ni oder Pt, besonders bevorzugt Pd, enthalten. Der oder die Katalysator(en) werden bevorzugt in Mengen von 0,05 Gew.-% bis 10 Gew.-%, besonders bevorzugt von 0,5 Gew.-% bis 5 Gew.-%, bezogen auf das Gesamtgewicht der zu kuppelnden Verbindungen eingesetzt.

Besonders geeignete Katalysatoren sind Komplexverbindungen von Metallen der VIII. Nebengruppe, insbesondere Komplexe des Palladium(0), die an Luft stabil sind, Pd-Komplexe die sich leicht mit Organometallreagenzien, wie z.B. Lithiumalkylen oder Organomagnesiumverbindungen, oder Phosphinen zu Palladium(0)-Komplexen reduzieren lassen, oder Palladium(2)-Komplexen gegebenenfalls unter Zusatz von PPh₃ oder anderen Phosphinen. Beispielsweise können PdCl₂(PPh₃)₂, PdBr₂(PPh₃)₂ oder Pd(OAc)₂ oder Mischungen aus diesen Verbindungen unter Zusatz von PPh₃ eingesetzt werden. Bevorzugt wird Pd(PPh₃)₄ ohne oder unter Zusatz von Phosphinen, in einer bevorzugten Ausführungsform ohne Zusatz von Phosphinen, eingesetzt, welches in preiswerter Form zur Verfügung steht. Als Phosphine werden bevorzugt PPh₃, PEtPh₂, PMePh₂, PEt₂Ph oder PEt₃, besonders bevorzugt PPh₃, eingesetzt..

Es ist jedoch auch möglich als Katalysatoren Palladiumverbindungen ohne Phosphinzusatz zu einzusetzen, beispielsweise Pd(OAc)₂.

Als Base sind beispielsweise Hydroxide, wie z.B. NaOH, KOH, LiOH, Ba(OH)₂, Ca(OH)₂, Alkoxide, wie z.B. NaOEt, KOEt, LiOEt, NaOMe, KOMe, LiOMe, Alkalimetallsalze von Carbonsäuren, wie z.B. Natrium-, Kalium- oder Lithiumcarbonat, -hydrogencarbonat, -acetat, -citrat, -acetylacetonat, -glycinat, oder andere Carbonate, wie z.B. Cs₂CO₃ oder Tl₂CO₃, Phosphate, wie z.B. Natriumphosphat, Kaliumphosphat oder Lithiumphosphat, oder Mischungen aus diesen eingesetzt werden. Bevorzugt wird Natriumcarbonat eingesetzt. Die Basen können als Lösungen in Wasser oder als Suspensionen in organischen Lösungsmitteln, wie Toluol, Dioxan oder DMF, eingesetzt werden. Bevorzugt sind Lösungen in Wasser, da die erhaltenen Produkte aufgrund ihrer geringen Löslichkeit in Wasser so vom Reaktionsgemisch leicht abgetrennt werden können.

Es ist auch möglich weitere Salze, beispielsweise LiCl oder LiBr, als Hilfsmittel einzusetzen.

Als organische Lösungsmittel kommen grundsätzlich alle Lösungsmittel oder Lösungsmittelgemische in Frage, welche mit Boronsäureestem nicht reagieren. Dies sind in der Regel Verbindungen, welche keine Halogenatome oder keine gegenüber Thiophen-Pinakolinboronsäureestern reaktive Wasserstoffatome aufweisen. Geeignete Lösungsmittel sind beispielsweise Alkane wie Pentan, Hexan und Heptan, Aromaten wie Benzol, Toluol und Xylole, Ethergruppen enthaltende Verbindungen wie Dioxan, Dimethoxyethan und Tetrahydrofuran und polare Lösungsmittel wie Dimethylformamid oder Dimethylsulfoxid. Bevorzugt werden im erfindungsgemäßen Verfahren Aromaten als Lösungsmittel eingesetzt. Ganz besonders bevorzugt ist Toluol. Es ist auch möglich, als Lösungsmittel Mischungen aus zwei oder mehreren dieser Lösungsmittel einzusetzen.

Die Aufarbeitung der Reaktionsmischung erfolgt nach an sich bekannten Verfahren, z.B. durch Verdünnen, Fällen, Filtration, Extraktion, Waschen, Rekristallisation aus geeigneten Lösungsmitteln, Chromatographie und/oder Sublimation. Beispielsweise kann eine Aufarbeitung in der Weise erfolgen, dass die Reaktionsmischung nach vervollständigter Reaktion in ein Gemisch aus saurem (Eis-)Wasser, z.B. hergestellt aus 1 molarer Salzsäure, und Toluol gegossen, die organische Phase abgetrennt, mit Wasser gewaschen, das als Feststoff enthaltene Produkt abfiltriert, mit Toluol gewaschen und anschließend im Vakuum getrocknet wird. Die Verbindungen der allgemeinen Formel (IV) und (V) können bereits ohne weitere anschließende Reinigungsprozesse in hoher Qualität und Reinheit erhalten werden und sind halbleitend. Es ist jedoch möglich, diese Produkte nach bekannten Verfahren, z.B. durch Rekristallisation, Chromatographie oder Sublimation weiter zu reinigen.

Mit dem Verfahren können die erfindungsgemäßen Verbindungen ohne aufwändige Reinigungsverfahren mit nur sehr geringen Mengen an Verunreinigungen hergestellt werden. Insbesondere werden nach diesem Verfahren die erfindungsgemäßen Verbindungen weitgehend frei von schwer abtrennbaren homologen Oligomeren mit höherem oder niedrigerem Molekulargewicht erhalten, so dass eine aufwändige Aufreinigung schwer trennbarer Gemische entfällt.

Die in diesem Verfahren eingesetzten bororganischen Verbindungen und Aryl- bzw. Heteroarylverbindungen, insbesondere der allgemeinen Formel (III), können nach bekannten Verfahren hergestellt werden oder sind käuflich zu erwerben. Die Herstellung der Pinakolinboronsäureseter der allgemeinen Formel (II) wird beispielsweise in Feast et al, J. Mater. Chem. 2003, 13, 1269-1273 beschrieben.

Die neuen Verbindungen der allgemeinen Formel (I) und insbesondere (I-a) und (I-a-2) bis (I-a-3) eigenen sich - nicht zuletzt aufgrund ihrer hohen Reinheit - hervorragend zur Herstellung von halbleitenden Schichten in elektronischen Bauteilen, wie beispielsweise Feldeffekt-Transistoren, organischen Lumineszenzdioden, photovoltaischen Zellen, Lasern oder Sensoren. Insbesondere die neuen Verbindungen der allgemeinen Formel (I), worin X für eine Alkoxysilylgruppe oder eine Siloxangruppe steht, bevorzugt die Verbindungen der allgemeinen Formeln (I-a-2) und (I-a-3), eignen sich zur Herstellung von Monoschichten, insbesondere selbstorganisierenden- Monoschichten (sog. SAMs), für Feldeffekt-Transistoren. So sind die neuen Verbindungen der allgemeinen Formel (I), worin X für eine Alkoxysilylgruppe oder eine Siloxangruppe steht, bevorzugt die Verbindungen der allgemeinen Formeln (I-a-2) und (I-a-3), bevorzugt geeignet zur Herstellung von halbleitenden Monoschichten, besonders bevorzugt jedoch solchen Monoschichten, die sowohl eine dünne Dielektrika-Schicht (Isolatorschicht) als auch eine dünne halbleitende Schicht enthalten. Es ist weiterhin möglich, auf die dünne halbleitende Schicht weitere halbleitende Schichten aufzutragen. Zudem ist es möglich, mit Hilfe der neuen Verbindungen der allgemeinen Formel (I), worin X für eine Alkoxysilylgruppe oder eine Siloxangruppe steht, bevorzugt mit Hilfe der Verbindungen der allgemeinen Formeln (I-a-2) und (I-a-3), solche Schichten zu erzeugen, die zwischen einer Diektrika-Schicht und einer weiteren halbleitenden Schicht liegen. Dies kann von Vorteil sein, da eine solche Zwischenschicht die Qualität der daraufliegenden Halbleiterschicht beispielsweise durch besseren Kontakt zwischen der Halbleiter- und Dielektrika-Schicht erhöht. Daraus resultieren beispielsweise bessere Eigenschaften von Feldeffekt-Transistoren, wie z.B. erhöhte Ladungträgermobilität, größeres On/Off-Ratio und niedrigere Einsatzspannung.

Weiterhin Gegenstand der vorliegenden Erfindung ist daher die Verwendung der erfindungsgemäßen Verbindungen zur Herstellung von halbleitenden Schichten für elektronische Bauteile, bevorzugt die Verwendung von neuen Verbindungen der allgemeinen Formel (I), worin X für eine Alkoxysilylgruppe oder eine Siloxangruppe steht, besonders bevorzugt von Verbindungen der allgemeinen Formeln (I-a-2) und (I-a-3), zur Herstellung von Monoschichten, die sowohl eine Dielektrika-Schicht (Isolatorschicht) als auch eine halbleitende Schicht enthalten. Ganz besonders bevorzugt können die Verbindungen der allgemeinen Formeln (I-a-2) und (I-a-3) zur Bildung selbstorganisierender Schichten (SAMs), d.h. als sogenannte SAM-Moleküle, verwendet werden.

Verbindungen der allgemeinen Formel (I), worin X für eine Alkoxysilylgruppe oder eine Siloxangruppe steht, besonders bevorzugt von Verbindungen der allgemeinen Formeln (I-a-2) und (I-a-3), lassen sich besonders gut auf Substrate oder Oberflächen, die im Wesentlichen aus Silizium oder oxidischen Materialien wie z.B. SiO₂, Al₂O₃, ZrO₂, HfO₂ oder Ta₂O₅ verankern. Die Verankerung auf diesen Substraten bzw. Oberflächen erfolgt dabei mit dem die Silyl- oder Siloxangruppe tragenden Ende der neuen Verbindungen. Die erfindungsgemäßen Verbindungen besitzen daher gegenüber bekannten thiolgruppenhaltigen SAM-Molekülen den Vorteil, auf anderen Oberflächen als kostspieligen Goldoberflächen verankert werden zu können. Damit sind die erfindungsgemäßen Verbindungen zudem anderen Anwendungen zugänglich als die bekannten thiolgruppenhaltigen SAM-Moleküle.

Die neuen Verbindungen der allgemeinen Formel (I), worin X für einen Vinylrest steht, insbesondere die Verbindungen der allgemeinen Formel (I-a-1), können an der Doppelbindung mit Hilfe einfacher chemischer Reaktionen, die dem Fachmann an sich bekannt sind, weiter modifiziert werden.

Als solche chemischen Reaktionen zur Modifizierung dieser bevorzugten Verbindungen kommen insbesondere solche Reaktionen in Betracht, bei denen gezielt und ausschließlich die Doppelbindung CH₂=CH- modifiziert, der übrige Teil der Verbindung jedoch nicht verändert wird. Beispielhaft für solche chemischen Reaktionen seien Polymerisations-, Oxidations-, Additions-oder Substitutionsreaktionen genannt. Bevorzugt sind Additions- und Polymerisationsreaktionen, besonderes bevorzugt sind Hydrosilylierungsreaktionen.

In einem ersten Schritt eine Verbindung der allgemeinen Formel (I), worin X für eine Vinylgruppe steht, herzustellen und in einem zweiten Schritt in eine Verbindung der allgemeinen Formel (I) umzuwandeln, in der X eine andere Bedeutung als Vinyl hat, kann besonders in den Fällen von Vorteil sein, in denen es sich bei der Gruppe X, die eine andere Bedeutung als Vinyl hat, um eine Gruppe handelt, die unter den Reaktionsbedingungen der Suzuki-Kupplung nicht stabil ist. Dies ist beispielsweise bei Chlorsilangruppen, Alkoxysilangruppen oder Siloxangruppen der Fall. Da die Ethenylgruppe gegenüber den Reaktionsbedingungen der Suzuki-Kupplung stabil ist und durch vorangehend aufgeführte einfache und dem Fachmann allgemein bekannt Reaktionen entsprechend modifiziert werden kann, ist dieser Weg insbesondere zur Herstellung von neuen Verbindungen der allgemeinen Formel (I), worin X für eine Alkoxysilyl- oder Siloxangruppe steht, bevorzugt solchen der allgemeinen Formeln (I-a-2) oder (I-a-3) bevorzugt.

Hydrosilylierungen und deren Durchführung sind dem Fachmann bekannt. Bevorzugt zur Modifizierung der neuen Verbindungen der allgemeinen Formel (I), worin X für einen Vinylrest steht, insbesondere die neuen Verbindungen der allgemeinen Formel (I-a-1), wird die Hydrosilylierung bei einer Temperatur von +20°C bis +200°C, bevorzugt von +30°C bis +120°C, besonderes bevorzugt von +60°C bis +90°C, in einem organischen Lösungsmittel oder Lösungsmittelgemisch mit einem metallhaltigen Katalysator durchgerührt.

Als metallhaltiger Katalysator können verschiedene Metall-Komplexe, insbesondere von Metallen der VIII. Nebengruppe des Periodensystems der Elemente, verwendet werden, beispielsweise Komplexe von Rh, Ir, Ni, Co, Pd, Pt und Ru. Bevorzugte Katalysatoren für die Hydrosilylierung sind Pt-Komplexe, wie beispielsweise H₂PtCl₆ z.B. in Isopropanol (Spier Katalysator) und Pt₂{[(CH₂=CH)Me₂Si]₂O}₃ oder ähnliche Verbindungen mit anderem Metall-Ligand Verhältnis (Karstedt Katalysatoren). Weiterhin bevorzugt sind Platin-cyclovinylmethylsiloxan-Komplexe aus cyclischen Methylvinylsiloxanen oder Platin-divinyltetramethylsiloxan-Komplexe in Xylol, die kommerziell erhältlich sind. Geeignete Mengen an Pt-Katalysator sind 1 ppm bis 1000 ppm, bevorzugt 5 ppm bis 100 ppm, besonders bevorzugt 10 bis 50 ppm Platin bezogen auf das Gesamtgewicht des Reaktionsgemisches.

Als organische Lösungsmittel für die Hydrosilylierung kommen im Prinzip sämtliche Lösungsmittel oder Lösungsmittelgemische in Frage, welche die Verbindungen der allgemeinen Formel (I), worin X für einen Vinylrest steht, insbesondere die neuen Verbindungen der allgemeinen Formel (I-a-1), bei Reaktionstemperatur gut lösen. Geeignete Lösungsmittel sind beispielsweise Alkane wie Hexan und Heptan, Aromaten wie Benzol, Toluol und Xylole, sowie Ethergruppen enthaltende Verbindungen wie Dioxan, Dimethoxyethan und Tetrahydrofuran. Bevorzugt werden in dem neuen Verfahren aromatische Lösungsmittel eingesetzt. Ganz besonders bevorzugt ist Toluol. Es ist allerdings auch möglich, als Lösungsmittel Mischungen aus zwei oder mehreren dieser Lösungsmittel einzusetzen.

Die neuen Verbindungen der allgemeinen Formel (I), worin X für einen Vinylrest steht, insbesondere die Verbindungen der allgemeinen Formel (I-a-1) eigenen sich daher sowohl zur Herstellung von Verbindungen, die geeignet sind selbstorganisierende Monoschichten, auch "self assembled monolayers" oder kurz "SAMs" genannt, zu bilden als auch zur Herstellung von halbleitenden Schichten für elektronische Bauteile insbesondere von SAM-Schichten.

Ebenfalls Gegenstand der vorliegenden Erfindung ist daher die Verwendung der neuen Verbindungen der allgemeinen Formel (I), worin X für einen Vinylrest steht, insbesondere der Verbindungen der allgemeinen Formel (I-a-1), zur Herstellung halbleitender Schichten für elektronische Bauteile insbesondere von SAM-Schichten sowie die Verwendung der neuen Verbindungen der allgemeinen Formel (I), worin X für einen Vinylrest steht, insbesondere der neuen Verbindungen der allgemeinen Formel (I-a-1), zur Herstellung von Verbindungen, die geeignet sind selbstorganisierende Monoschichten, auch "self assembled monolayers" oder kurz "SAMs" genannt, zu bilden.

Zur Verwendung für die Herstellung von halbleitenden Schichten werden die neuen Verbindungen der allgemeinen Formeln (I), (I-a) oder (I-a-1) bis (I-a-3) bevorzugt in Form von Schichten auf geeignete Substrate, zum Beispiel auf mit elektrischen oder elektronischen Strukturen versehene Silizium-Wafer, Polymerfolien oder Glasscheiben aufgetragen. Für den Auftrag kommen prinzipiell sämtliche dem Fachmann bekannten Auftragsverfahren in Betracht. Beispielsweise können die erfindungsgemäßen Verbindungen aus der Gasphase oder aus Lösung aufgetragen werden, wobei man das Lösungsmittel anschließend verdampft. Das Aufbringen aus Lösung kann nach den bekannten Verfahren, beispielsweise durch Sprühen, Tauchen, Drucken und Rakeln, Spin-Coating und durch Ink-Jet-Druck erfolgen. Bevorzugt werden die erfindungsgemäßen Verbindungen aus der flüssigen Phase, z.B. durch Spin-Coating aus einem geeigneten Lösungsmittel, z.B. Toluol, aufgebracht.

Gegenstand der vorliegenden Erfindung sind daher weiterhin Schichten, bevorzugt im Wesentlichen bestehend aus Verbindungen der allgemeinen Formel (I), insbesondere (I-a) oder bevorzugt (I-a-1) bis (I-a-3).

Bevorzugt sind dies Monoschichten, besonders bevorzugt SAMs (self assembled monolayers), d.h solche, die aus selbstorganisierenden erfindungsgemäßen Verbindungen hergestellt wurden.

In bevorzugten Ausführungsformen sind dies Schichten aus Verbindungen der allgemeinen Formel (I), worin X für eine Alkoxysilyl- oder Siloxangruppe steht, insbesondere aus Verbindungen der allgemeinen Formel (I-a-2) oder (I-a-3), die in besonders bevorzugten Ausführungsformen Monoschichten bilden, die sowohl eine Dielektrika-Schicht (Isolatorschicht) als auch eine halbleitende Schicht enthalten.

Die erfindungsgemäßen Schichten können nach dem Aufbringen weiter modifiziert werden, beispielsweise durch eine Temperatürbehandlung, z.B. unter Durchlaufen einer flüssigkristallinen Phase, oder zur Strukturierung z.B. durch Laserablation.

Die erfindungsgemäßen Schichten, die aus den erfindungsgemäßen Verbindungen hergestellt werden, zeichnen sich durch hohe Reinheit und folglich geringe Defekte aus. Insbesondere SAMs, d.h. aus selbstorganisierenden erfindungsgemäßen Verbindungen, bevorzugt denen der allgemeinen Formel (I-a-2) oder (I-a-3), hergestellte Schichten, haben den Vorteil, dass sie extrem dünn, d.h. in molekularen Dimensionen von maximal wenigen Nanometern, bevorzugt monomolekular, in einfacher Weise sowohl eine Dielektrika-Schicht als auch eine halbleitende Schicht bilden. Durch den Einsatz solcher SAMs kann beispielsweise die Schwellenspannung eines Feldeffekt-Transistors erniedrigt werden.

Die erfindungsgemäßen Schichten sind für den Einsatz in aktiven und lichtemittierenden elektronischen Bauteile wie Feldeffekt-Transistoren, organischen Lumineszenzdioden, photovoltaischen Zellen, Lasern oder Sensoren geeignet.

Weiterhin Gegenstand der vorliegenden Erfindung sind daher solche elektronische Bauteile enthaltend wenigstens eine der erfindungsgemäßen Schichten.

### Beispiele

5-(10-Undecenyl)-2,2'-bithiophen, 5-Bromo-5'-Ethyl-2,2'-bithiophen und 5-Bromo-5"-ethyl-2,2':5',2"-terthiophen wurden nach bekannten Verfahren hergestellt (Synthesis, 1993, S.1099; Chem. Mater., 1993, Band 5, S. 430; J. Mater. Chem. 2003, Band 13, S. 197).

Alle Reaktionsgefäße wurden vor Gebrauch nach üblicher Schutzgastechnik ausgeheizt und mit Stickstoff geflutet.

### Beispiel 1: Herstellung von 4,4,5,5-Tetramethyl-2-[5'-(10-undecenyl)-2,2'-bithien-5-yl]-1,3,2-dioxaborolan (II-a)

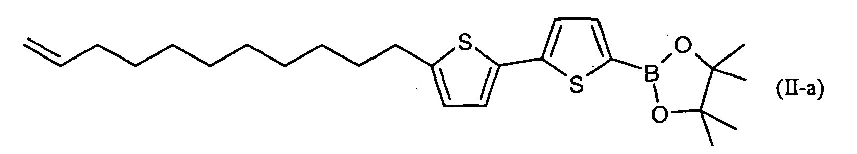

70 ml wasserfreies Tetrahydrofuran (THF) wurden mittels Trockeneis/Aceton auf -74°C heruntergekühlt. Hierzu wurden mit einer Spritze 5.6 ml einer 2.5 M Butyllithiumlösung in Hexan zugetropft. Anschließend wurde ein homogenes Gemisch aus 5-(10-Undecenyl)-2,2'-bithiophen (4.46 g, 14 mmol) in 120 ml wasserfreiem THF zugetropft und 30 min bei -74° nachgerührt. Das Kältebad wurde entfernt, so dass die Temperatur ansteigt. Bei ca. 0°C wurde der Reaktionsansatz wieder heruntergekühlt und bei -74°C 2-Isopropoxy-4,4,5,5-tetramethyl-1,3,2-dioxaborolan (3.3 ml, 16 mmol) über einer Spritze zugegeben. Es wurde 30 min bei -74°C nachgerührt, das Kühlbad erneut entfernt und die Temperatur auf 20°C ansteigen gelassen. Das Reaktionsgemisch wurde in 200 ml eisgekühltes Wasser - gemischt mit 15 ml 1 M HCl - gegeben und mit 500 ml Diethylether extrahiert. Die Etherphase wurde abgetrennt, mit Wasser gewaschen, über Na₂SO₄ getrocknet, filtriert und das Lösungsmittel vollständig am Rotationsverdampfer entfernt. Ausbeute: 6.03 g (97 % der Theorie) dunkelblaues, kristallines (II-a).

GC MS Analyse: M^{•+} 99%, m/e = 444.

¹H NMR (CDCl₃, TMS/ppm): 1.22-1.45 (overlapped peaks with a max at 1.283, 14H), 1.345 (s, 12 H), 1.672 (m, J=7.5 Hz, M = 5, 2H), 2.037 (q, J=7.2 Hz, 2H), 2.781 (t, J=7.3 Hz, 2H), 4.928 (d, J=10.3 Hz, 1H), 4.991 (d, J=17.1 Hz, 1H), 5.811 (m, 1H), 6.676 (d, J=3.4 Hz, 1H), 7.037 (d, J=3.9 Hz, 1H), 7.152 (d, J=3.9 Hz, 1H), 7.496 (d, J=3.4 Hz, 1H).

### Beispiel 2: Herstellung von 5-Ethyl-5"'-(10-undecenyl)-2,2':5',2":5",2"'-quaterthiophen (I-a-1.1)

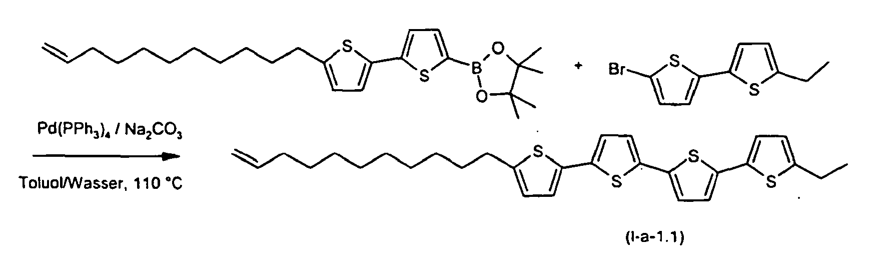

5-Bromo-5'-ethyl-2,2'-bithiophen (3.28 g, 12 mmol) wurde vorgelegt und unter Schutzgas Tetrakis(thiphenylphosphin)palladium Pd(PPh₃)₄ (675 mg, 0.6 mmol) dazugegeben. Es wurde eine Lösung von 4,4,5,5-Tetramethyl-2-[5'-(10-undecenyl)-2,2'-bithien-5-yl]-1,3,2-dioxaborolane (I-a) (6.01 g, 12 mmol) in 120 ml wasserfreiem Toluol und 18 ml einer 2 M wässrigen Na₂CO₃-Lösung vorbereitet und mit Stickstoff desoxidiert. Beide Lösungen wurden nacheinander mit Spritzen zum Reaktionsansatz gegeben und das Reaktionsgemisch anschließend für 20 Stunden unter Rückfluss erhitzt. Das Reaktionsgemisch wurde nach Abkühlen in ein Gemisch aus 200 ml Wasser, 80 ml 1 M HCl und 300 ml Toluol gegeben. Die organische Phase wurde abgetrennt, mit Wasser gewaschen, über MgSO₄ getrocknet, filtriert und das Lösungsmittel vollständig am Rotationsverdampfer entfernt. Das olivgrüne, feste Produkt wurde in n-Hexan umkristallisiert. Ausbeute: 4.18 g (68 % der Theorie) gelbes Pulver (I-a-1.1).

FD MS Analyse: M^{•+} 100%, m/e = 510.0

¹H NMR (CDCl₃, TMS/ppm): 1.23 - 1.44 (overlapped peaks with max at 1.288, 15 H, including t at 1.326 ppm, J= 7.6, 3H), 1.680 (m, J= 7.5, M=5, 2H), 2.040 (q, J=7.5, 2H), 2.789 (t, J=7.6, 2H), 2.840 (q, J=7.2, 2H), 4.928 (d, J=10.3, 1H), 4.989 (d, J=17.1, 1H), 5.813 (m, 1H), 6.679 (d, J=3.4, 1H), 6.699 (d, J=3.4, 1H), 6.98 (overlapped peaks, 4H), 7.030 (d, J=3.4, 2H).

### Beispiel 3: Herstellung von 5-Ethyl-5""-(10-undecenyl)-2,2':5',2":5",2"':5"' 2""-quinquethiophen (I-a-1.2)

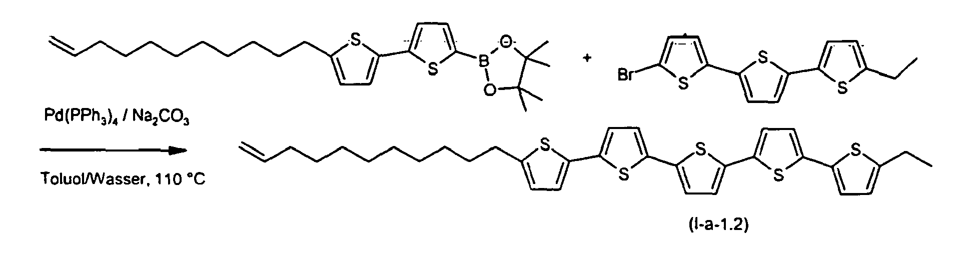

5-Ethyl-5""-(10-undecenyl)-2,2':5',2":5",2"':5"',2"''-quinquethiophen (IV-a-2) wurde aus 5-Bromo-5"-ethyl-2,2':5',2"-terthiophen (2.13 g, 6 mmol), Pd(PPh₃)₄ (350 mg, 0.3 mmol) und 4,4,5,5-Tetramethyl-2-[5'-(10-undecenyl)-2,2'-bithien-5-yl]-1,3,2-dioxaborolan (I-a) (3.11 g, 7 mmol) wie in Beispiel 2 beschrieben in 40 ml wasserfreiem Toluol und 10 ml einer 2 M wässrigen Na₂CO₃-Lösung hergestellt. Nach Aufarbeitung wie in Beispiel 2 wurde die organische Phase über einen Glassfilter G4 filtriert und der orangefarbene Niederschlag in Toluol umkristallisiert. Ausbeute: 2.96 g (83 % der Theorie) dunkelorangefarbenes Pulver (I-a-1.2).

FD MS Analyse: M^{•+} 100%, m/e = 592.1

H NMR (CDCl₃, TMS/ppm): 1.23 - 1.44 (overlapped peaks with max at 1.290, 15 H, including t at 1.329 ppm, J= 7.3, 3H), 1.682 (m, J= 7.5, M=5, 2H), 2.041 (q, J=7.0, 2H), 2.791 (t, J=7.6, 2H), 2.841 (q, J=7.6, 2H), 4.930 (d, J=10.3, 1H), 4.989 (d, J=17.1, 1H), 5.813 (m, 1H), 6.684 (d, J=3.9, 1H), 6.704 (d, J=3.4, 1H), 6.99 (overlapped peaks, 4H), 7.048 (d, J=3.4, 2H), 7.053 (s, 2H).

Schmelzverhalten (°C): K 240 N 242 I (K = kristallin, N = nematisch flüssigkristallin, I = isotrop flüssig).

### Beispiel 4: Herstellung von 5-Ethyl-5"'-(11-triethoxysilylundecyl)-2,2':5',2":5"',2"'-quaterthiophen (I-a-2.1)

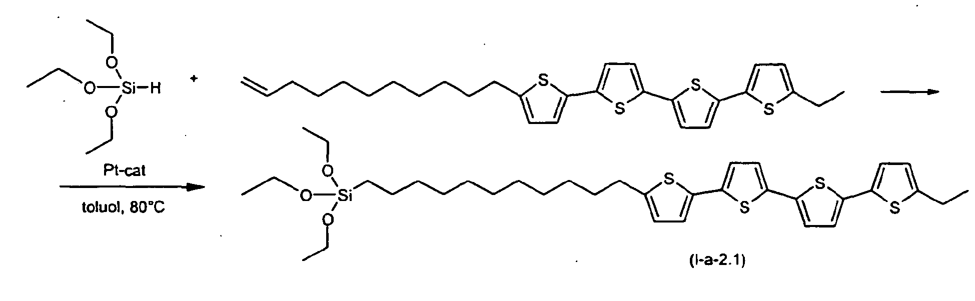

5-Ethyl-5"'-(10-undecenyl)-2,2':5',2":5",2"'-quaterthiophen (I-a-1.1) aus Beispiel 2 (410 mg, 0.8 mmol) wurde im 100 ml Mehrhalskolben mit Kühler, Gasableitung, Thermometer und Septum-Adapter vorgelegt und mit Stickstoff geflutet. Dann wurden 15 ml wasserfreies Toluol dazu gegeben und das Gemisch auf 80°C erhitzt bis alles (I-a-1.1) gelöst war. Anschließend wurden Triethoxysilane (324 mg, 2 mmol) und eine Lösung eines Platincyclovinylmethylsiloxane-Komplexes cyclischer Methylvinylsiloxane (3-3,5 Gew. % Pt, 5 ml, bspw. erhältlich über ABCR) wurden mittels Spritzen nacheinander bei 80°C zugegeben. Das Gemisch wurde für 20 Stunden bei 80°C gerührt und anschließend nach Abkühlung das Lösungsmittel am Rotationsverdampfer entfernt. Das erhaltene feste, gelbe Produkt wurde über Kieselgel in Toluol filtriert. Ausbeute: 151 mg (27 % der Theorie) gelbes Pulver (I-a-2.1).

FD MS Analyse: M^{•+} 100%, m/e = 674.2

¹H NMR (CDCl₃, TMS/ppm): 0.628 (t, J=8.1 Hz, 2H), 1.225 (t, J=7.1 Hz, 9H), 1.23 - 1.44 (overlapped peaks with max at 1.267, 21 H, including t at 1.327 ppm, J= 7.6 Hz, 3H), 1.678 (m, J= 7.3, M=5, 2H), 2.788 (t, J=7.6 Hz, 2H), 2.839 (q, J=8.3 Hz, 2H), 3.814 (q, J=6.9 Hz, 6H), 6.680 (d, J=3.4 Hz, 1H), 6.700 (d, J=3.4 Hz, 1H), 6.98 (overlapped peaks, 4H), 7.030 (d, J=3.9 Hz, 2H)

Schmelzverhalten (°C): K 160 1 (K = kristallin, I = isotrop flüssig).

### Beispiel 5: Herstellung von 1-[11-(5'''-ethyl-2,2':5',2'':5'',2'''-quaterthien-5-yl)undecyl]-1,1,3,3-tetramethyldisiloxan (I-a-3.1)

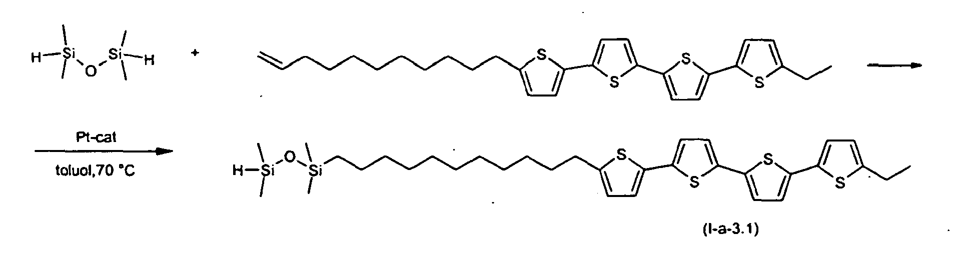

1-[11-(5"'-ethyl-2,2':5',2":5",2"'-quaterthien-5-yl)undecyl]-1,1,3,3-tetramethyldisiloxan (I-a-3.1) wurde aus 5-Ethyl-5"'-(10-undecenyl)-2,2':5',2":5",2"'-quaterthiophen (I-a-1.1) (1.79 g, 3.5 mmol), 1,1,3,3-Tetamethyldisiloxane (25 ml, 18.8 g, 140 mmol) und einer Lösung eines Platincyclovinylmethylsiloxane-Komplexes cyclische Methylvinylsiloxane (3-3,5 Gew. % Pt, 10 ml) wie in Beispiel 4 beschrieben in 70 ml wasserfreiem Toluol bei 70°C hergestellt und gereinigt. Ausbeute: 2.26 g (94 % der Theorie) gelbes Pulver (I-a-3.1).

FD MS Analyse: M^{•+} 99+%, m/e = 644.0

¹H NMR (CDCl₃, TMS/ppm): 0.056 (s, 6H), 0.160 (d, J=2.5 Hz, 6H), 0.527 (t, J=7.6 Hz, 2H), 1.23 - 1.43 (overlapped peaks with max at 1.267, 21 H, including t at 1.326 ppm, J= 7.8 Hz, 3H), 1.680 (m, J= 7.5, M=5, 2H), 2.789 (t, J=7.6 Hz, 2H), 2.840 (q, J=7.2 Hz, 2H), 4.676 (m, J=2.8 Hz, M=7, 1H), 6.679 (d, J=3.4 Hz, 1H), 6.699 (d, J=3.4 Hz, 1H), 6.98 (overlapped peaks, 4H), 7.029 (d, J=3.9 Hz, 2H).

## Patentansprüche

1. Verbindungen der allgemeinen Formel (I), 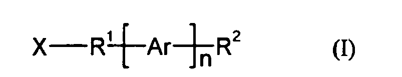 worin
n eine ganze Zahl von 4 bis 10 ist,
Ar unabhängig für jedes n für einen gegebenenfalls substituierten 1,4-Phenylen-, 2,7-Fluorenylen-, 2,5-Thienylen- oder 1,2-Ethenylenrest steht, wobei wenigstens ein gegebenenfalls substituierter 2,5-Thienylenrest oder gegebenenfalls substituierter 2,5-Thienylenrest und gegebenenfalls substituierter 1,4-Phenylenrest enthalten ist,
R¹ für eine gegebenenfalls durch ein(e) oder mehrere O- oder S-Atome, Silylen-, Phosphonoyl- oder Phosphorylgruppen unterbrochene C₃-C₃₀-Alkylengruppe steht,
R² für H oder eine lineare oder verzweigte C₁-C₂₀-Alkylgruppe, bevorzugt eine C₁-C₁₂-Alkylgruppe, oder eine gegebenenfalls durch ein(e) oder mehrere O- oder S-Atome, Silylen-, Phosphonoyl- oder Phosphorylgruppen unterbrochene lineare C₁-C₂₀-Alkylgruppe, bevorzugt C₁-C₁₂-Alkylgruppe, steht und
X für eine Gruppe ausgewählt aus einer gegebenenfalls substituierten Vinyl-, Alkoxysilyl-, Chlorosilyl- oder Siloxangruppe steht.

2. Verbindungen gemäß Anspruch 1, **dadurch gekennzeichnet, dass** Ar für gegebenenfalls substituiertes 2,5-Thienylen steht.

3. Verbindungen gemäß Anspruch 1 oder 2, **dadurch gekennzeichnet, dass** X für eine Alkoxysilylgruppe oder eine Siloxangruppe steht.

4. Verbindungen gemäß Anspruch 1 oder 2, **dadurch gekennzeichnet, dass** X für eine Vinylgruppe steht.

5. Verfahren zur Herstellung von Verbindungen gemäß wenigstens einem der Ansprüche 1 bis 4, **dadurch gekennzeichnet, dass** eine bororganische Verbindung mittels Suzuki-Kupplung mit einem Aryl- bzw. Heteroarylhalogenid umgesetzt wird.

6. Verfahren gemäß Anspruch 5, **dadurch gekennzeichnet, dass** als bororganische Verbindung eine Verbindung der allgemeinen Formel (II), 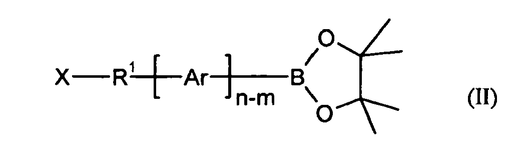 worin n, Ar, R¹ und X die in Anspruch 1 genannte Bedeutung haben und m für eine ganze Zahl von 1 bis 5 steht,
und als Aryl- bzw. Heteroarylhalogenid eine Verbindung der allgemeinen Formel (III), 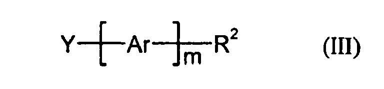 worin m und Ar die für die allgemeine Formel (II) und R² die in Anspruch 1 genannte Bedeutung haben und Y für Cl, Br, I oder -O-SO₂-R³, wobei R³ für eine Methyl-, Trifluormethyl-, Phenyl- oder Tolylgruppe steht,
eingesetzt und diese miteinander umgesetzt werden.

7. Verfahren zur Herstellung von Verbindungen gemäß Anspruch 3, **dadurch gekennzeichnet, dass** eine Verbindung der allgemeinen Formel (I), worin X für eine Vinylgruppe steht, hydrosilyliert wird.

8. Verwendung von Verbindungen gemäß wenigstens einem der Ansprüche 1 bis 4 zur Herstellung von halbleitenden Schichten für elektronische Bauteile.

9. Verwendung von Verbindungen gemäß wenigstens einem der Ansprüche 1 bis 4 zur Herstellung von Monoschichten, die sowohl die Funktion einer Dielektrika-Schicht (Isolatorschicht) als auch die Funktion einer halbleitenden Schicht haben.

10. Halbleitende Schichten, **dadurch gekennzeichnet, dass** sie Verbindungen gemäß wenigstens einem der Ansprüche 1 bis 4 enthalten.

11. Schichten gemäß Anspruch 10, **dadurch gekennzeichnet, dass** es sich um Monoschichten handelt, die sowohl die Funktion einer Dielektrika-Schicht (Isolatorschicht) als auch einer halbleitenden Schicht haben.

12. Elektronisches Bauteil enthaltend wenigstens eine Schicht gemäß wenigstens einem der Ansprüche 10 oder 11.

## Claims

1. Compounds of the general formula (I) 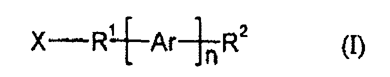 where
n is a whole number from 4 to 10,
Ar is, independently for each n, an optionally substituted 1,4-phenylene, 2,7-fluorenylene, 2,5-thienylene or 1,2-ethenylene radical, with the proviso that at least an optionally substituted 2,5-thienylene radical or optionally substituted 2,5-thienylene radical and optionally substituted 1,4-phenylene radical is present,
R¹ is a C₃-C₃₀-alkylene group optionally interrupted by one or more oxygen or sulphur atoms, silylene, phosphonoyl or phosphoryl groups,
R² is H or a linear or branched C₁-C₂₀-alkyl group, preferably a C₁-C₁₂-alkyl group, or a linear C₁-C₂₀-alkyl group, preferably C₁-C₁₂-alkyl group, optionally interrupted by one or more oxygen or sulphur atoms, silylene, phosphonoyl or phosphoryl groups, and
X is a group selected from an optionally substituted vinyl, alkoxysilyl, chlorosilyl or siloxane group.

2. Compounds according to Claim 1, **characterized in that** Ar is optionally substituted 2,5-thienylene.

3. Compounds according to Claim 1 or 2, **characterized in that** X is an alkoxysilyl group or a siloxane group.

4. Compounds according to Claim 1 or 2, **characterized in that** X is a vinyl group.

5. Process for producing compounds according to at least one of the Claims 1 to 4, **characterized in that** an organoboron compound is reacted with an aryl or heteroaryl halide by means of Suzuki coupling.

6. Process according to Claim 5, **characterized in that** the organoboron compound used is a compound of the general formula (II) 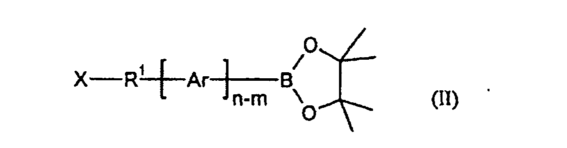 where n, Ar, R¹ and X are each as defined in Claim 1 and m is a whole number from 1 to 5,
and the aryl or heteroaryl halide used is a compound of the general formula (III) 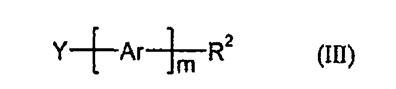 where m and Ar are each as defined for the general formula (II) and R² is as defined in Claim 1 and Y is Cl, Br, I or -O-SO₂-R³, where R³ is a methyl, trifluoromethyl, phenyl or tolyl group,
and these compounds are reacted with each other.

7. Process for producing compounds according to Claim 3, **characterized in that** a compound of the general formula (I) where X is a vinyl group is hydrosilylated.

8. Use of compounds according to at least one of the Claims 1 to 4 for producing semiconductive layers for electronic components.

9. Use of compounds according to at least one of the Claims 1 to 4 for producing monolayers having not only the function of a dielectric layer (insulator layer) but also the function of a semiconductive layer.

10. Semiconductive layers, **characterized in that** they comprise compounds according to at least one of the Claims 1 to 4.

11. Layers according to Claim 10, **characterized in that** they comprise monolayers having not only the function of a dielectric layer (insulator layer) but also the function of a semiconductive layer.

12. Electronic component comprising at least one layer according to at least one of the Claims 10 and 11.

## Revendications

1. Composés de formule générale (I), 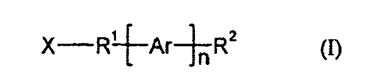 où
n vaut un nombre entier de 4 à 10,
Ar représente, indépendamment pour chaque n, un radical 1,4-phénylène, 2,7-fluoroénylène, 2,5-thiénylène ou 1,2-éthénylène, le cas échéant substitué, au moins un radical 2,5-thiénylène le cas échéant substitué ou au moins un radical 2,5-thiénylène le cas échéant substitué et au moins un radical 1,4-phénylène étant contenu,
R¹ représente un groupement alkylène en C₃-C₃₀ le cas échéant interrompu par un ou plusieurs atomes O ou S, groupements silylène, phosphonoyle ou phosphoryle,
R² représente H ou un groupement alkyle en C₁-C₂₀ linéaire ou ramifié, de préférence un groupement alkyle en C₁-C₁₂ ou un groupement alkyle en C₁-C₂₀, de préférence un groupement alkyle en C₁-C₁₂, linéaire le cas échéant interrompu par un ou plusieurs atomes O ou S, groupements silylène, phosphonoyle ou phosphoryle et
X représente un groupement choisi parmi un groupement vinyle, alcoxysilyle, chlorosilyle ou siloxane, le cas échéant substitué.

2. Composés selon la revendication 1, **caractérisés en ce que** Ar représente un groupement 2,5-thiénylène le cas échéant substitué.

3. Composés selon la revendication 1 ou 2, **caractérisés en ce que** X représente un groupement alcoxysilyle ou un groupement siloxane.

4. Composés selon la revendication 1 ou 2, **caractérisés en ce que** X représente un groupement vinyle.

5. Procédé pour la préparation de composés selon au moins l'une quelconque des revendications 1 à 4, **caractérisé en ce qu'**un composé organique du bore est transformé au moyen d'un couplage de Suzuki avec un halogénure d'aryle ou, selon le cas, d'hétéroaryle.

6. Procédé selon la revendication 5, **caractérisé en ce qu'**on utilise comme composé organique du bore un composé de formule générale (II), 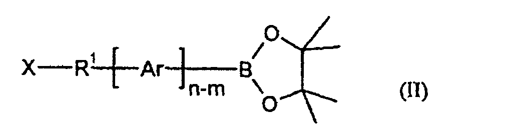 où n, Ar, R¹ et X ont la signification mentionnée dans la revendication 1 et m représente un nombre entier de 1 à 5,
et comme halogénure d'aryle ou, selon le cas, d'hétéroaryle un composé de formule générale (III), 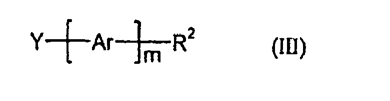 où m et Ar ont la signification mentionnée pour la formule générale (II) et R² a la signification mentionnée dans la revendication 1 et Y représente Cl, Br, I ou -O-SO₂-R³, R³ représentant méthyle, trifluorométhyle, phényle ou tolyle,
et on les transforme l'un avec l'autre.

7. Procédé pour la préparation de composés selon la revendication 3, **caractérisé en ce qu'**on hydrosilyle un composé de formule générale (I), dans laquelle X représente un groupement vinyle.

8. Utilisation de composés selon au moins l'une quelconque des revendications 1 à 4 pour la préparation de couches semi-conductrices pour des pièces électroniques.

9. Utilisation de composés selon au moins l'une quelconque des revendications 1 à 4 pour la préparation de monocouches, qui ont la fonction d'une couche diélectrique (couche isolante) ainsi que la fonction d'une couche semi-conductrice.

10. Couches semi-conductrices, **caractérisées en ce qu'**elles contiennent des composés selon au moins l'une quelconque des revendications 1 à 4.

11. Couches selon la revendication 10, **caractérisées en ce qu'**il s'agit de monocouches, qui présentent la fonction d'une couche diélectrique (couche isolante) ainsi que d'une couche semi-conductrice.

12. Pièce électronique contenant au moins une couche selon au moins l'une quelconque des revendications 10 ou 11.
